# EUROPÄISCHE PATENTSCHRIFT

(11) **EP 0 592 896 B1**
(45) Veröffentlichungstag und Bekanntmachung des Hinweises auf die Patenterteilung: **17.01.2001**
(21) Anmeldenummer: 93115898.4
(22) Anmeldetag: 01.10.1993
(51) Int. Cl.: C07D 213/61, C07D 213/64, C07D 213/73

(54) **Verfahren zur Herstellung von 2-substituierten 5-Alkylpyridinen**
Process for the preparation of 2-substituted 5-alkyl-pyridines
Procédé pour la préparation de méthyl-5 pyridines substitués en 2

(30) Priorität: 14.10.1992 DE 4234637
(43) Veröffentlichungstag der Anmeldung: 20.04.1994
(73) Patentinhaber: BAYER AG, 51368 Leverkusen (DE)
(72) Erfinder: Kraus, Helmut, Dr., D-51519 Odenthal (DE); Klausener, Alexander, Dr., D-50670 Köln (DE); Fürstenwerth, Hauke, Dr., D-51381 Leverkusen (DE)

(56) Entgegenhaltungen:
- EP-A- 0 060 071
- DD-A- 240 010
- CHEMICAL ABSTRACTS, vol. 97, no. 21, 22. November 1982, Columbus, Ohio, US; abstract no. 182171q, A. R. KATRITZKY ET AL. 'Novel ring opening of aminoheterocycles: facile syntheses of omega-alkylaminopentadienenitriles' Seite 805 ;

## Beschreibung

Die Erfindung betrifft ein Verfahren zur Herstellung von 2-substituierten 5-Alkyl-pyridinen durch Umsetzung von aminomethylenierten 2-Pentensäurederivaten mit Säuren oder Aminen. Die Erfindung betrifft weiterhin die Herstellung der genannten aminomethylenierten 2-Pentensäurederivate.

2-substituierte 5-Methyl-pyridine sind wichtige Zwischenprodukte zur Herstellung von Herbiziden (EP 483), Insektiziden (EP 235 725) und Pharmazeutika (CA 1.189.509).

Als einfachste Methode zur Synthese von 2-substituierten 5-Methyl-pyridinen erscheint prinzipiell die direkte Funktionalisierung von β-Picolin. Die Umsetzung mit Natriumamid in flüssigem Ammoniak nach Tschitschibabin ergibt jedoch neben dem gewünschten Isomeren auch einen großen Teil an 2-Amino-3-methyl-pyridin (US 4 386 209). Die Chlorierung von β-Picolin-N-oxid mit speziellen Chlorierungsmitteln führt in guter Ausbeute zum 2-Chlor-5-methylpyridin; die Durchführung dieser Reaktion ist jedoch recht aufwendig (DE-OS 38 00 179 und DE-OS 38 39 332). Eine Hydroxylierung wurde bisher nur zur Herstellung von 6-Hydroxy-nicotinsäure beschrieben (EP 152 949); die dazu erforderliche Reduktion der Carboxylgruppe ist jedoch umständlich. Dagegen wird die Umwandlung von 2-Amino- bzw. 2-Hydroxy-pyridinen zu den Chlorderivaten als recht gut durchführbar beschrieben (GB 1.215.387; EP 72 777).

Bei Heterocyclen-Synthesen wird oft der Ringaufbau einer Substitution am Grundgerüst vorgezogen. Eine interessante 3 + 3-Verknüpfung wird in EP 108 483 beschrieben; durch Umsetzung von Morpholinopropen mit Acrylsäurederivaten kommt man jedoch nur zum entsprechenden Dihydropyridin, das anschließend aufwendig aromatisiert werden muß. Die Verwendung von α-Chlor-acrylnitril gemäß EP 162 464 führt direkt, jedoch in geringer Ausbeute zum 2-Chlor-5-methyl-pyridin.

Die 5 + 1-Verknüpfung, d.h. die Umsetzung von Propylidencyanessigestern mit Dimethylformamid(DMF)-Acetal und anschließende Cyclisierung mit Bromwasserstoff wird als eine bequeme Möglichkeit zur Herstellung 5-substituierter 2-Brom-pyridine (J. Org. Chem. 43, 2529 (1978)) und 2-Chlor-pyridine (JP 55/76 863 (1980)) beschrieben, wobei jedoch das Erfordernis besteht, daß die 3- oder 5-Position mit negativierenden I-Substituenten besetzt ist.

Angesichts dieses Standes der Technik ist es überraschend, daß nicht-substituierte, aminomethylenierte 2-Pentensäurederivate zu 2-substituierten 5-Alkylpyridinen cyclisiert werden können.

Auch die Herstellung der genannten aminomethylenierten 2-Pentensäurederivate als Zwischenprodukte aus nicht aminomethylenierten 2-Pentensäurederivaten der unten genannten Art und ortho-Amiden gelingt überraschend glatt, da aufgrund der Molekülstruktur eine Aminomethylenierung gleichermaßen in der erwünschten γ-Position wie in der unerwünschten α-Position zu erwarten war. Des weiteren waren Isomerisierung, Dimerisierung und Polymerisation des 2-Pentensäurederivats selbst zu erwarten; so ist in J. Org. Chem. 46 (1981), 3795 die alkalische Dimerisierung von substituierten Acrylestern beschrieben; in Coll. Czech. Chem. Commun. Vol. 35, (1970), 1224 ist die Isomerisierung und Dimerisierung von ungesättigten Nitrilen und in J. Org. Chem. 47 (1982), 163 die bevorzugte Reaktion von konjugierten Acrylestern an der α-Position unter Doppelbindungsverschiebung beschrieben.

Die EP-A-60 071 betrifft u.a. 5-phenyl substituierte Pyridine und ein Verfahren zu ihrer Herstellung durch Umsetzung von 5-Dimethylamino-4-phenyl-2.4-pentadiennitrilen mit Ammoniak. Die Reaktion verläuft auch unter relativ drastischen Bedingungen mit so geringer Ausbeute, daß sie für eine wirtschaftliche Nutzung ungeeignet erscheint.

Die DD-A 240 010 beschreibt substituierte 2-lmino-1H-pyridin-1-amine und ein Verfahren zu ihrer Herstellung. Substituierte aromatische Pyridine sind auf diese Weise nicht zugänglich.

Die Erfindung betrifft ein Verfahren zur Herstellung von 2-substituierten 5-Alkyl-pyridinen der Formel 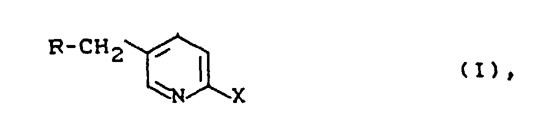 in der
- R: für Wasserstoff oder geradkettiges oder verzweigtes C₁-C₄-Alkyl steht und
- X: Chlor, Brom, Hydroxy oder Amino bedeutet,
das dadurch gekennzeichnet ist, daß man aminomethylenierte 2-Pentensäurederivate der Formel 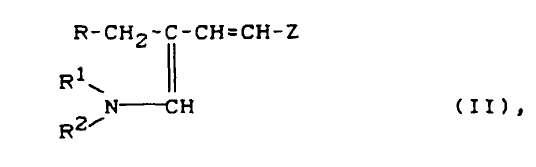 in der
- R¹ und R²: unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R¹ und R² gemeinsam mit dem N-Atom, das sie substituieren, einen 5- bis 8-gliedrigen Ring bilden können, der ein weiteres Heteroatom aus der Gruppe von N, O und S enthalten kann, und
- Z: für CN oder COOR³ steht, wobei R³ geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, bedeutet,
in Gegenwart oder in Abwesenheit eines polaren Lösungsmittels bei -10°C bis +25°C in Gegenwart von 1 bis 10 Mol, bezogen auf das 2-Pentensäurederivat, von HCl, HBr, einer konzentrierten starken anorganischen oder organischen nicht oxydierenden Sauerstoffsäure oder in Gegenwart von 1 bis 10 Mol, bezogen auf das Butenderivat, von NH₃ cyclisiert.

Die Erfindung betrifft weiterhin in bevorzugter Weise die Herstellung der 2-substituierten 5-Alkyl-pyridine, das dadurch gekennzeichnet ist, daß ein aminomethyleniertes 2-Pentensäurederivat eingesetzt wird, das erhalten wird bei der Umsetzung von nicht aminomethylenierten 2-Pentensäurederivaten der Formel

R-CH₂-CH₂-CH=CH-Z (III)

bzw.

R-CH₂-CH=CH-CH₂-Z (IV)

mit ortho-Amiden der Formel 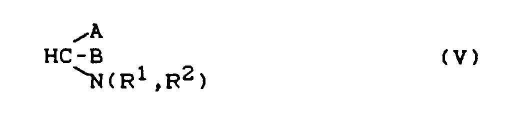 wobei in den Formeln
- R, Z, R¹ und R²: den oben genannten Bedeutungsumfang haben und
- A und B: unabhängig voneinander für OR⁴, OR⁵, N(R⁶,R⁷) oder N(R⁸,R⁹) stehen, worin R⁴, R⁵, R⁶, R⁷, R⁸ und R⁹ unabhängig voneinander die Bedeutung von R¹ bzw. R² haben,
wobei die Umsetzung in Gegenwart oder in Abwesenheit eines Lösungsmittels, dessen Acidität geringer ist als die C-H-Acidität des 2-Pentensäurederivats, bei 50 bis 200°C, 0,01 bis 10 bar und bei einem Molverhältnis von 2-Pentensäurederivat : ortho-Amid = 1 bis 50 : 1 durchgeführt wird.

Geradkettiges oder verzweigtes C₁-C₈-Alkyl ist beispielsweise Methyl, Ethyl, Propyl, Isopropyl, Butyl, Isobutyl, tert.-Butyl, eines der isomeren Pentyle, Hexyle oder Octyle, bevorzugt einer der genannten C₁-C₄-Alkylreste.

Geradkettiges oder verzweigtes C₂-C₈-Alkenyl ist beispielsweise Vinyl, Propenyl, Allyl, eines der isomeren Butenyle, Pentenyle, Hexenyle oder Octenyle, bevorzugt einer der genannten C₃-C₄-Alkenylreste.

Geradkettiges oder verzweigtes C₂-C₈-Alkoxyalkyl ist beispielsweise Methoxymethyl, Ethoxymethyl oder ein weiterer Rest aus der Gruppe C₃-C₉-Alkyl, in welchem eine CH₂-Gruppe durch ein O-Atom ersetzt ist.

Geradkettiges oder verzweigtes C₃-C₈-Alkoxyalkenyl ist beispielsweise Methoxyvinyl, Ethoxyvinyl, Methoxyallyl, 2-Methoxy-propenyl oder ein anderer aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methylcyclopropyl, Dimethyl-cyclopropyl, Cyclobutyl, Methylcyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl oder ein weiterer dem Fachmann bekannter Rest dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrrolin, Pyrazol, Imidazol, Thiazol, Oxazol, Pyridin, Pyrimidin, Piperazin, Morpholin, Pyran, Azepin, Azocin, Isoxazol, Isothiazol, Pyridazin und Pyrazin, die am N-Atom durch C₁-C₄-Alkyl oder durch Hydroxy-C₁-C₄-alkyl substituiert sein können.

Es ist dem Fachmann bekannt, daß ungesättigte heterocyclische Ringe einen mehr oder weniger stark ausgeprägten aromatischen Charakter haben können. In bevorzugter Weise seien als solche heterocyclische Ringe Morpholin, Pyrrolidin und Piperidin genannt, die durch C₁-C₄-Alkyl oder durch Hydroxy-C₁-C₄-alkyl substituiert sein können.

Weiterhin können R¹ und R² gemeinsam mit dem N-Atom, das sie substituieren, einen 5- bis 8-gliedrigen gesättigten oder ungesättigten Ring bilden, der ein weiteres Heteroatom aus der Gruppe N, O und S enthalten kann. Solche Ringe sind beispielsweise die oben genannten Heterocyclen.

R ist in bevorzugter Weise Wasserstoff.

In weiterhin bevorzugter Weise treten an die Stelle von R¹ und R² die Substituenten R¹¹ bzw. R¹², die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹¹ und R¹² gemeinsam mit dem N-Atom, das sie substituieren, einen 5- bis 6-gliedrigen Ring bilden können, der ein weiteres Heteroatom aus der Gruppe von N, O und S enthalten kann.

In besonders bevorzugter Weise treten an die Stelle von R¹¹ und R¹² die Substituenten R²¹ bzw. R²², die unabhängig voneinander geradkettiges oder verzweigtes C₁-C₄-alkyl bedeuten, wobei weiterhin R²¹ und R²² gemeinsam mit dem N-Atom, das sie substituieren, Morpholin, Pyrrolidin oder Piperidin bedeuten können, die durch C₁-C₄-Alkyl oder durch Hydroxy-C₁-C₄-alkyl substituiert sein können.

Unter die erfindungsgemäß einsetzbaren ortho-Amide fallen DMF-Acetale, Aminalester und Triaminomethane der Formeln

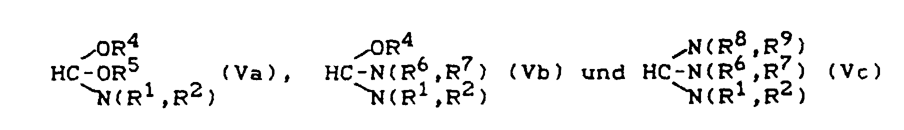

Die erfindungsgemäßen Reaktionen können beispielhaft wie folgt dargestellt werden:

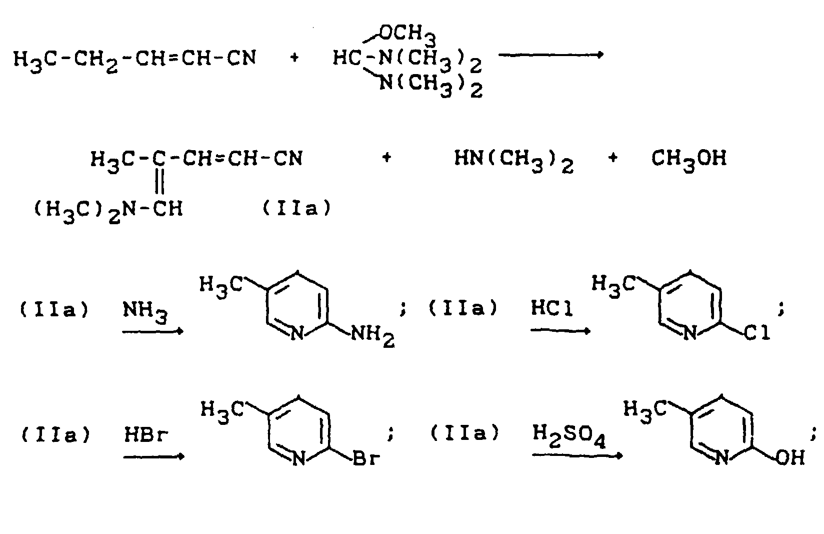

Hierbei ist die Cyclisierung mit Hilfe von Ammoniak, Chlorwasserstoff, Bromwasserstoff bzw. Schwefelsäure dargestellt. Setzt man anstelle der Cyanogruppe (für Z) die Carbonsäureestergruppe ein und benutzt zur Cyclisierung Ammoniak, so erhält man formelmäßig den folgenden beispielhaften Reaktionsverlauf:

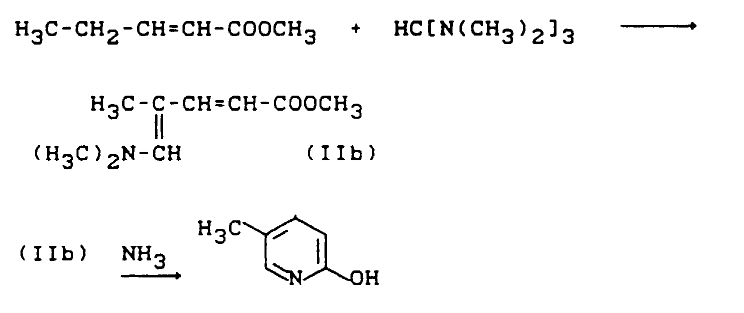

Die Aminomethylenierung kann in allen Lösungsmitteln durchgeführt werden, deren Acidität geringer ist als die des umzusetzenden 2-Pentensäurederivats (III) bzw. (IV); die Aminomethylenierung mit Hilfe von DMF-Acetal gelingt weiterhin auch in Alkoholen als Lösungsmittel. Es ist jedoch vorteilhaft, ohne Lösungsmittel zu arbeiten.

Das 2-Pentensäurederivat wird mindestens im äquimolaren Verhältnis, bezogen auf das ortho-Amid eingesetzt. In bevorzugter Weise wird das 2-Pentensäurederivat jedoch im molaren Überschuß eingesetzt, um das teure ortho-Amid möglichst vollständig zu nutzen. Für den Fall, daß ohne Lösungsmittel gearbeitet wird, wird in einem sehr hohen molaren Überschuß des 2-Pentensäurederivats gearbeitet, welches nach der Aminomethylenierung zurückgewonnen werden kann. Als molares Verhältnis ergibt sich somit allgemein eine Menge von 1 bis 50 Mol 2-Pentensäurederivat pro 1 Mol ortho-Amid.

Die Aminomethylenierung wird bei einer Temperatur von 50 bis 200°C und einem Druck von 0,01 bis 10 bar, bevorzugt 0,1 bis 3 bar, besonders bevorzugt bei 1 bar, durchgeführt. Beispielsweise wird bei Siedetemperatur des 2-Pentensäurederivates gearbeitet. Es hat sich als vorteilhaft erwiesen, das 2-Pentensäurederivat vorzulegen, auf die gewünschte Reaktionstemperatur zu bringen und dann das ortho-Amid im Maße seines Verbrauchs zuzugeben; dadurch liegt es stets nur in geringer Konzentration vor und gibt wenig Anlaß zu Nebenreaktionen. Die hierbei entstehenden leicht flüchtigen Produkte (Amine, Alkohole) werden simultan abdestilliert. Für den Fall, daß das eingesetzte 2-Pentensäurederivat und das ortho-Amid nahe beieinanderliegende Siedepunkte haben, kann beispielsweise das ortho-Amid in die Mitte einer Füllkörperkolonne eingespeist werden, die sich über einem Sumpf von siedenden 2-Pentensäurederivat befindet. Bei einer solchen Verfahrensweise wird das ortho-Amid mit dem 2-Pentensäurederivat innerhalb der Kolonne umgesetzt; etwa vorhandene Selbstkondensationsprodukte des 2-Pentensäurederivats bleiben im Sumpf und stehen zur Umsetzung mit dem ortho-Amid nicht zur Verfügung, wodurch die Bildung von Nebenprodukten weiter abgesenkt wird. Diese Verfahrensweise ist in überraschender Weise günstig, um das bevorzugte γ-substituierte Propenderivat gegenüber dem unerwünschten α-substituierten zu bevorzugen.

Nach Beendigung der Reaktion wird das gewünschte aminomethylenierte Reaktionsprodukt durch Vakuumdestillation des Reaktionssumpfes gewonnen. Für die nachfolgende Cyclisierung kann auf eine weitere Reinigung verzichtet werden.

Das γ-aminomethylenierte 2-Pentensäurederivat, bevorzugt in seiner rohen Form, wird zur Cyclisierung mit einem Überschuß, beispielsweise einem solchen von 1 bis 10 Mol, an Chlorwasserstoff, Bromwasserstoff, einer konzentrierten starken anorganischen der organischen, nicht oxidierenden Sauerstoffsäure oder von NH₃ umgesetzt. Hierzu kann ein polares Lösungsmittel herangezogen werden, beispielsweise Essigsäure, Ameisensäure, Chloroform, Carbonsäureamide oder Alkohole. Die Cyclisierung wird bei einer Temperatur von -10°C bis +25°C, bevorzugt bei 0 bis 10°C durchgeführt. Beispielsweise wird das aminomethylenierte 2-Pentensäurederivat, in Substanz oder gelöst in einem der genannten Lösungsmittel, zu einer Lösung von HCl oder HBr in Eisessig getropft. Ebenso ist eine umgekehrte Dosierung möglich. Man kann auch HCl- bzw. HBr-Gas in das aminomethylenierte 2-Pentensäurederivat einleiten, wobei sich dieses vorteilhafterweise gelöst in einem der genannten Lösungsmittel befindet. HCl bzw. HBr werden in bevorzugter Weise in einem molaren Überschuß von 1,5 bis 5 verwendet. Durch analoge Anwendung nicht oxidierender Sauerstoffsäuren der anorganischen oder organischen Reihe kann im Sinne des obigen Reaktionsschemas die Hydroxylgruppe als X-Substituent eingeführt werden. Analoge Anwendung von NH₃ führt zur Einführung von Amino als X-Substituent. Sauerstoffsäuren, die hierfür geeignet sind, sind beispielsweise Schwefelsäure, Phosphorsäure, Benzolsulfonsäure, p-Toluolsulfonsäure und andere dem Fachmann bekannte. Die Umsetzung mit NH₃ zum 2-Amino-pyridin erfolgt in bevorzugter Weise in Alkoholen, wie Methanol, Ethanol, Propanol oder Butanol. Ein Zusatz von Alkoholat, z.B. Na-methylat kann the Reaktivität von NH₃ erhöhen.

### Beispiele

### Beispiel 1

50 ml 98,7 %iges cis-2-Pentennitril aus der Destillation von 80 %iger technischer Ware (enthält 1,1 % 2-Methylbutennitril-2) wurden in einem 3-Hals-Kolben zum Rückfluß erhitzt. Dann wurden langsam 12,8 g 97,3 %iger Methylaminalester (Rest DMF) zugetropft. Man erhitzte noch 2 h unter Rückfluß, zog am Rotationsverdampfer die leichtflüchtigen Bestandteile ab und führte mit dem Rückstand eine Kugelrohrdestillation durch.

Das Destillat (19,7 g) wurde in 100 ml Eisessig gelöst, und bei 11°C wurde trockenes HCl-Gas eingeleitet. Nach 1 h bei 5°C ließ man den Ansatz auf Raumtemperatur kommen und über Nacht stehen. Nach dem Einengen und der Zugabe von Toluol und Wasser stellte man auf pH 7-8. Die organische Phase wurde abgetrennt, eingeengt und destilliert. Man erhielt 2-Chlor-5-methylpyridin in 27,6 % der theoretischen Ausbeute als 98,6 %ige Ware. Das Produkt wurde durch GC-(Retentionszeit) und GC-MS-Vergleich mit authentischer Ware identifiziert. ¹H-NMR (CDCl₃); 2,3 d (CH₃), 7,22 (H³), 7,48 (H⁴), 8,62 (H⁶) ppm.

### Beispiel 2

23 g trans-2-Pentensäuremethylester wurden analog Beispiel 1 mit 0,1 Mol Methylaminalester aminomethyieniert und der Rückstand der Kugelrohrdestillation in 100 ml Methanol gelöst. Man setzte noch 20 ml 30 %ige Natriummethylatlösung zu, leitete 15 g Ammoniakgas ein und erhitzte 20 h unter Rückfluß. Anschließend wurde mit 100 ml Wasser verdünnt und mit konzentrierter Salzsäure auf pH 4 gestellt. HPLC-Analytik ergab, daß 2-Hydroxy-5-methylpyridin in 42,6 % d.Th. entstanden war.

### Beispiel 3

In einem 500 ml Einhalskolben mit aufgesetzter 1 m-Füllkörperkolonne und Kolonnenkopf wurden 120 g trans-3-Pentennitril und 0,3 g Hydrochinonmonomethylether als Stabilisator in 100 ml Isododecan vorgelegt und unter Rückfluß erhitzt. In der Mitte der Kolonne wurden mit Hilfe einer Dosierpumpe innerhalb von 2 h 30 g Ethylaminalester zugegeben. Am Kopf der Kolonne wurden leichtflüchtige Komponenten abdestilliert. Der Sumpf wurde eingeengt, wobei überschüssiges Pentennitril zurückgewonnen wurde, und einer Kugelrohrdestillation unterworfen. Der Anteil an "dimerem" Pentennitril war höher als in Beispiel 1, der Anteil an aminomethylenierten "Dimeren" jedoch von 12,3 auf 3,3 % der theoretischen Ausbeute (bezogen auf Aminalester) gesunken.

Die Umsetzung mit HCl/Eisessig analog Beispiel 1 ergab 38,9 % 2-Chlor-5-methylpyridin.

### Beispiel 4

Analog Beispiel 3 wurde cis-2-Pentennitril mit Methylaminalester aminomethyleniert. Der Rückstand der Kugelrohrdestillation wurde mit 20 ml Eisessig verdünnt und zu 300 ml einer 30 %igen Lösung von HBr in Eisessig getropft. Nach der Aufarbeitung wurden 43,3 % der theoretischen Ausbeute an 2-Brom-5-methylpyridin erhalten.

### Beispiel 5

Analog Beispiel 4 wurde der Rückstand mit 20 ml konzentrierter Schwefelsäure versetzt, auf Wasser gegossen, und nach Aufarbeitung konnte 2-Hydroxy-5-methylpyridin als Hauptprodukt identifiziert werden.

### Beispiel 6

Analog Beispiel 1 wurde cis-2-Pentennitril mit DMF-Acetal aminomethyleniert. Nach Umsetzung mit HCl/Eisessig konnte 2-Chlor-5-methylpyridin als Hauptprodukt identifiziert werden.

### Beispiel 7

Analog Beispiel 3 wurde cis-2-Pentennitril mit Tris(dimethylamino)methan, das in das obere Drittel der Kolonne eingepumpt wurde, aminomethyleniert.

Der eingeengte Rückstand wurde mit Eisessig verdünnt und bei 5°C zu einer gesättigten Lösung von HCl in Eisessig getropft. Nach der üblichen Aufarbeitung wurde das Produkt in 43,6 % der theoretischen Ausbeute erhalten.

### Beispiel 8

Rohes aminomethyleniertes cis-2-Pentennitril aus Beispiel 7 wurde in THF gelöst und bei 0°C mit wäßriger 30 %iger Ammoniaklösung versetzt. Nach 6 h Kochen am Rückfluß war 2-Amino-5-methylpyridin entstanden.

### Beispiel 9

Rohes aminomethyleniertes cis-2-Pentennitril aus Beispiel 7 wurde bei -10°C zu einer gesättigten Lösung von HCl-Gas in Chloroform/Eisessig getropft. Durch GC-Analyse mit internem Standard konnte gezeigt werden, daß 2-Chlor-5-methlypyridin in 48,7 % der theoretischen Ausbeute, bezogen auf Tris(dimethylamino)methan, entstanden war.

## Patentansprüche

1. Verfahren zur Herstellung von 2-substituierten 5-Alkyl-pyridinen der Formel 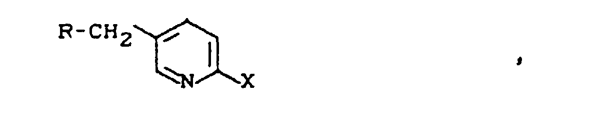 in der
R für Wasserstoff oder geradkettiges oder verzweigtes C₁-C₄-Alkyl steht und
X Chlor, Brom, Hydroxy oder Amino bedeutet,
dadurch gekennzeichnet, daß man aminomethylenierte 2-Pentensäurederivate der Formel 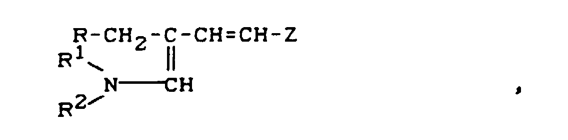 in der
R¹ und R² unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R¹ und R² gemeinsam mit dem N-Atom, das sie substituieren, einen 5- bis 8-gliedrigen Ring bilden können, der ein weiteres Heteroatom aus der Gruppe von N, O und S enthalten kann, und
Z für CN oder COOR³ steht, wobei R³ geradkettiges oder verzweigtes C₁-C₈-Alkyl, C₂-C₈-Alkenyl, C₂-C₈-Alkoxyalkyl, C₂-C₈-Alkoxyalkenyl, C₃-C₈-Cycloalkyl, bedeutet,
in Gegenwart oder in Abwesenheit eines polaren Lösungsmittels bei -10°C bis +25°C in Gegenwart von 1 bis 10 Mol, bezogen auf das 2-Pentensäurederivat, von HCl, HBr, einer konzentrierten starken anorganischen oder organischen nicht oxydierenden Sauer stoffsäure oder in Gegenwart von 1 bis 10 Mol, bezogen auf das Butenderivat, von NH₃ cyclisiert.

2. Verfahren nach Anspruch 1, dadurch gekennzeichnet, daß ein aminomethyleniertes 2-Pentensäurederivat eingesetzt wird, das erhalten wird bei der Umsetzung von nicht aminomethylenierten 2-Pentensäurederivaten der Formel
R-CH₂-CH₂-CH=CH-Z
bzw.
R-CH₂-CH=CH-CH₂-Z
mit ortho-Amiden der Formel 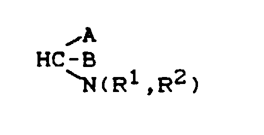 wobei in den Formeln
R, Z, R¹ und R² den in Anspruch 1 genannten Bedeutungsumfang haben und
A und B unabhängig voneinander für OR⁴, OR⁵, N(R⁶,R⁷) oder N(R⁸,R⁹) stehen, worin R⁴, R⁵, R⁶, R⁷, R⁸ und R⁹ unabhängig voneinander die Bedeutung von R¹ bzw. R² haben,
wobei die Umsetzung in Gegenwart oder in Abwesenheit eines Lösungsmittels, dessen Acidität geringer ist als die C-H-Acidität des 2-Pentensäurederivats, bei 50 bis 200°C, 0,01 bis 10 bar und bei einem Molverhältnis von 2-Pentensäurederivat : ortho-Amid = 1 bis 50 : 1 durchgeführt wird.

3. Verfahren nach Anspruch 1, dadurch gekennzeichnet, daß R die Bedeutung Wasserstoff annimmt.

4. Verfahren nach Anspruch 1, dadurch gekennzeichnet, daß an die Stelle von R¹ und R² die Substituenten R¹¹ bzw. R¹² treten, die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹¹ und R¹² gemeinsam mit dem N-Atom, das sie substituieren, einen 5- bis 8-gliedrigen Ring bilden können, der ein weiteres Heteroatom aus der Gruppe von N, O und S enthalten kann.

5. Verfahren nach Anspruch 4, dadurch gekennzeichnet, daß an die Stelle von R¹¹ und R¹² die Substituenten R²¹ bzw. R²² treten, die unabhängig voneinander geradkettiges oder verzweigtes C₁-C₄-Alkyl bedeuten, wobei weiterhin R²¹ und R²² gemeinsam mit dem N-Atom, das sie substituieren, Morpholin, Pyrrolidin oder Piperidin bedeuten können, die durch C₁-C₄-Alkyl oder durch Hydroxy-C₁-C₄-alkyl substituiert sein können.

6. Verfahren nach Anspruch 2, dadurch gekennzeichnet, daß bei Siedetemperatur des 2-Pentensäurederivates gearbeitet wird und die Reaktion in einer aufgesetzten Kolonne durch Einspeisung des ortho-Amids in diese Kolonne durchgeführt wird.

7. Verfahren nach Anspruch 2, dadurch gekennzeichnet, daß das rohe Aminomethylenierungsprodukt zur Cyclisierung eingesetzt wird.

## Claims

1. Process for the preparation of 2-substituted 5-alkyl-pyridines of the formula 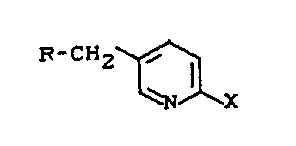 in which
R represents hydrogen or straight-chain or branched C₁-C₄-alkyl and
X denotes chlorine, bromine, hydroxyl or amino,
characterised in that aminomethylenated 2-pentenoic acid derivatives of the formula 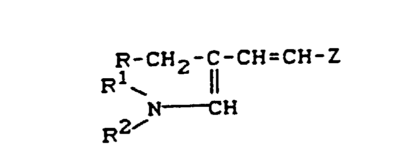 in which
R¹ and R² independently of one another represent straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₆-C₁₂-aryl, C₇-C₁₀-aralkyl or a 5- to 8-membered saturated or unsaturated heterocyclic ring, heteroatoms 1 or 2 of which are from the group consisting of N, O and S, where R¹ and R², together with the N atom which they substitute, can additionally form a 5- to 8-membered ring which can contain a further heteroatom from the group consisting of N, O and S, and
Z represents CN or COOR³, where R³ denotes straight-chain or branched C₁-C₈-alkyl, C₂-C₈-alkenyl, C₂-C₈-alkoxyalkyl, C₃-C₈-alkoxyalkenyl or C₃-C₈-cycloalkyl,
are cyclised in the presence or in the absence of a polar solvent at -10°C to +25°C in the presence of 1 to 10 mol, relative to the 2-pentenoic acid derivative, of HCl, HBr, a concentrated strong inorganic or organic non-oxidising oxygen acid or in the presence of 1 to 10 mol, relative to the butene derivative, of NH₃.

2. Process according to Claim 1, characterised in that an aminomethylenated 2-pentenoic acid derivative is employed which is obtained in the reaction of non-aminomethylenated 2-pentenoic acid derivatives of the formula
R-CH₂-CH₂-CH=CH-Z
or
R-CH₂-CH=CH-CH₂-Z
with ortho-amides of the formula 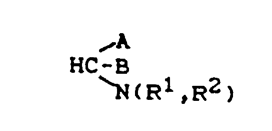 where in the formulae
R, Z, R¹ and R² have the scope of meaning mentioned in Claim 1 and
A and B independently of one another represent OR⁴, OR⁵, N(R⁶,R⁷) or N(R⁸,R⁹), wherein R⁴, R⁵, R⁶, R⁷, R⁸ and R⁹ independently of one another have the meaning of R¹ or R²,
the reaction being carried out in the presence or in the absence of a solvent whose acidity is lower than the C-H acidity of the 2-pentenoic acid derivative, at 50 to 200°C, 0.01 to 10 bar and at a molar ratio of 2-pentenoic acid derivative : ortho-amide = 1 to 50 : 1.

3. Process according to Claim 1, characterised in that R assumes the meaning hydrogen.

4. Process according to Claim 1, characterised in that the substituents R¹¹ and R¹² which independently of one another denote straight-chain or branched C₁-C₈-alkyl, cyclopropyl, cyclopentyl, cyclohexyl, phenyl or benzyl take the place of R¹ and R², it furthermore being possible for R¹¹ and R¹², together with the N atom which they substitute, to form a 5-to 8-membered ring which can contain a further heteroatom from the group consisting of N, O and S.

5. Process according to Claim 4, characterised in that the substituents R²¹ and R²² which independently of one another denote straight-chain or branched C₁-C₄-alkyl take the place of R¹¹ and R¹², it furthermore being possible for R²¹ and R²², together with the N atom which they substitute, to denote morpholine, pyrrolidine or piperidine, which can be substituted by C₁-C₄-alkyl or by hydroxy-C₁-C₄-alkyl.

6. Process according to Claim 2, characterised in that it is carried out at the boiling point of the 2-pentenoic acid derivative and the reaction is carried out in an attached column by feeding the ortho-amide into this column.

7. Process according to Claim 2, characterised in that the crude aminomethylenation product is employed for the cyclisation.

## Revendications

1. Procédé pour la préparation de 5-alkylpyridines substituées en position 2 répondant à la formule 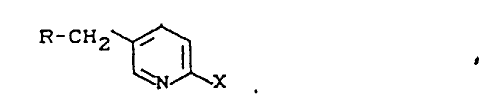 dans laquelle
R représente un atome d'hydrogène ou un groupe alkyle en C₁-C₄ à chaîne droite ou ramifiée, et
X représente un atome de chlore, un atome de brome, un groupe hydroxyle ou un groupe amino,
caractérisé en ce qu'on soumet à une cyclisation des dérivés aminométhyléniques d'acide 2-penténoïque répondant à la formule 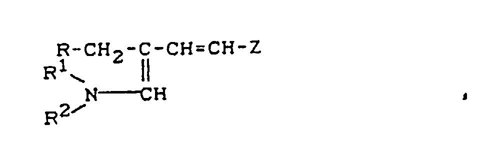 dans laquelle
R¹ et R² représentent, indépendamment l'un de l'autre, un groupe alkyle en C₁-C₈ à chaîne droite ou ramifiée, un groupe alcényle en C₂-C₈, un groupe alcoxyalkyle en C₂-C₈, un groupe alcoxyalcényle en C₃-C₈, un groupe cycloalkyle en C₃-C₈, un groupe aryle en C₆-C₁₂, un groupe aralkyle en C₇-C₁₀ ou encore un noyau hétérocyclique penta- à octogonal saturé ou insaturé dont les hétéroatomes représentent 1 ou 2 atomes choisis parmi le groupe comprenant un atome d'azote, un atome d'oxygène et un atome de soufre; en outre, R¹ et R² peuvent former ensemble avec l'atome d'azote qu'ils substituent un noyau penta- à octogonal qui peut contenir un hétéroatome supplémentaire choisi parmi le groupe comprenant un atome d'azote, un atome d'oxygène et un atome de soufre, et
Z représente un groupe CN ou un groupe COOR³ où R³ représente un groupe alkyle en C₁-C₈ à chaîne droite ou ramifiée, un groupe alcényle en C₂-C₈, un groupe alcoxyalkyle en C₂-C₈, un groupe alcoxyalcényle en C₃-C₈, un groupe cycloalkyle en C₃-C₈,
en présence ou en l'absence d'un solvant polaire, à une température de -10°C à +25°C, en présence de 1 à 10 moles, rapportées au dérivé d'acide 2-penténoïque, de HCl, de HBr, d'un acide oxygéné concentré, fort, inorganique ou organique, non oxydant, ou encore en présence de 1 à 10 moles, rapportés au dérivé de butène, de NH₃.

2. Procédé selon la revendication 1, caractérisé en ce qu'on met en oeuvre un dérivé aminométhylénique d'acide 2-penténoïque que l'on obtient lors de la mise en réaction de dérivés non aminométhyléniques d'acide 2-penténoïque répondant à la formule
R-CH₂-CH₂-CH=CH-Z,
respectivement
R-CH₂-CH=CH-CH₂-Z
avec des ortho-amides répondant à la formule 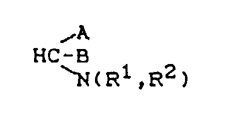 formules dans lesquelles
R, Z, R¹ et R² ont la portée de signification indiquée à la revendication 1, et
A et B représentent, indépendamment l'un de l'autre, un groupe OR⁴, un groupe OR⁵, un groupe N(R⁶,R⁷) ou un groupe N(R⁸,R⁹) dans lesquels R⁴, R⁵, R⁶, R⁷, R⁸ et R⁹ ont, indépendamment l'un de l'autre, la signification de R¹, respectivement de R²,
la mise en réaction ayant lieu en présence ou en l'absence d'un solvant dont l'acidité est inférieure à l'acidité C-H du dérivé d'acide 2-penténoïque, à une température de 50 à 200°C, sous une pression de 0,01 à 10 bar, et à un rapport molaire du dérivé d'acide 2-penténoïque : ortho-amide = 1 à 50 : 1.

3. Procédé selon la revendication 1, caractérisé en ce que R prend la signification d'un atome d'hydrogène.

4. Procédé selon la revendication 1, caractérisé en ce qu'on utilise, à la place de R¹ et de R², les substituants R¹¹ respectivement R¹² qui représentent, indépendamment l'un de l'autre, un groupe alkyle en C₁-C₈ à chaîne droite ou ramifiée, un groupe cyclopropyle, un groupe cyclopentyle, un groupe cyclohexyle, un groupe phényle ou un groupe benzyle; en outre, R¹¹ et R¹² peuvent former ensemble avec l'atome d'azote qu'ils substituent un noyau penta- à octogonal qui peut contenir un hétéroatome supplémentaire choisi parmi le groupe comprenant un atome d'azote, un atome d'oxygène et un atome de soufre.

5. Procédé selon la revendication 4, caractérisé en ce qu'on utilise, à la place de R¹¹ et de R¹², les substituants R²¹ respectivement R²² qui représentent, indépendamment l'un de l'autre, un groupe alkyle en C₁-C₄ à chaîne droite ou ramifiée; en outre, R²¹ et R²² peuvent représenter ensemble avec l'atome d'azote qu'ils substituent un groupe morpholino, un groupe pyrrolidino ou un groupe pipéridino qui peuvent porter un ou plusieurs substituants alkyle en C₁-C₄ ou hydroxyalkyle en C₁-C₄ identiques ou différents.

6. Procédé selon la revendication 2, caractérisé en ce qu'on travaille à la température d'ébullition du dérivé d'acide 2-penténoïque et on effectue la réaction dans une colonne superposée, par introduction de l'ortho-amide dans cette colonne.

7. Procédé selon la revendication 2, caractérisé en ce qu'on met en oeuvre pour la cyclisation le produit brut d'aminométhylénation.
